# EUROPEAN PATENT APPLICATION

(11) **EP 4 053 762 A1**
(43) Date of publication of application: **07.09.2022**
(21) Application number: 20882371.6
(22) Date of filing: 22.10.2020
(51) Int. Cl.: G06Q 10/04, G06Q 50/10

(54) **DISEASE PREDICTION SYSTEM, INSURANCE FEE CALCULATION SYSTEM, AND DISEASE PREDICTION METHOD**

(30) Priority: 28.10.2019 JP 2019195404
(71) Applicant: Anicom Holdings, Inc., Shinjuku-ku Tokyo 160-0023 (JP)
(72) Inventor: KOMORI, Nobuaki, Tokyo 160-0023 (JP); KAMEI, Tatsuhiko, Tokyo 160-0023 (JP); KAWAMOTO, Kousuke, Tokyo 160-0023 (JP); KIKUCHI, Ryou, Tokyo 160-0023 (JP); KISHIDA, Shigefumi, Tokyo 160-0023 (JP); IBE, Kunihiko, Tokyo 160-0023 (JP); SAIGUSA, Ryota, Tokyo 160-0023 (JP)
(74) Representative: Gill Jennings & Every LLP
(86) International application number: PCT/JP2020/039795
(87) International publication number: WO 2021/085313

(57) **Abstract**

An objective of the present invention is to provide a disease prediction system for assessing, by means of a simple method, the possibility that an animal may contract a disease in the future, and the like. The disease prediction system is provided with a reception means for receiving an input of a facial image of an animal excluding a human and an assessment means for outputting, by using a learned model, a prediction regarding contraction of a disease by the animal as inferred from the facial image of the animal that has been input to the reception means, the disease prediction system being characterized in that the learned model is a learned model that performs learning by using facial images of animals excluding humans and the presence or absence of a contracted disease within a predetermined period from the time of imaging the animals as training data, inputs a facial image of an animal, and outputs a prediction regarding whether the animal may contract a disease.

## Description

### TECHNICAL FIELD

The present invention relates to a disease prediction system, an insurance fee calculation system, and a disease prediction method. Specifically, the invention relates to a disease prediction system, an insurance fee calculation system, and a disease prediction method for providing information related to the future disease possibility of an animal based on a facial image of an animal.

### BACKGROUND ART

Pets such as dogs, cats or rabbits, and livestock such as cattle and pigs, are irreplaceable to humans. In recent years, while the average life of animals raised by humans has been greatly extended, animals more often contract any disease in their lives, and there is a problem of an increase in the medical expenses borne by owners.

In order to maintain the health of an animal, physical condition management through daily meals, exercise and the like and quick action in response to disorder is important. An animal, however, is unable to appeal the disorder of the body by its own words, and thus, the owner is aware of a disease of the animal for the first time when a sign that can be observed externally has appeared with progressing symptom presentation.

Thus, there is required a means for learning on, by means of a simple method, the possibility that an animal may contract a disease in the future. In particular, it is useful to learn the possibility of a future disease of an animal while it is in a state without any symptom or in a state it has not yet contracted a disease, since specific measures can be taken to prevent a disease.

Patent Document 1 discloses a computer system for diagnosing a subject, characterized by including: a first image acquisition means for acquiring a plurality of first subject images accompanied by a chronological change of the subject; a first image analysis means for performing image analysis of the first subject images that have been acquired; a second image acquisition means for acquiring a plurality of second subject images accompanied by a chronological change of another subject in the past; a second image analysis means for performing image analysis of the second subject images that have been acquired; a collating means for collating an image analysis result of the first subject images with a result of image analysis of the second subject images; and a diagnostic means for diagnosing the subject based on the result of collation.

Patent Document 1, however, relates to a system for diagnosing a subject, not for assessing the possibility that a subject may contract a disease in the future.

### RELATED ART DOCUMENT

### PATENT DOCUMENT

[Patent Document 1] WO 2018/211688

### SUMMARY OF THE INVENTION

### PROBLEMS TO BE SOLVED BY THE INVENTION

The invention aims to provide a disease prediction system for assessing, by means of a simple method, the possibility that an animal may contract a disease in the future, and the like.

### MEANS FOR SOLVING THE PROBLEMS

In an insurance company for operating health insurance targeting an animal, so-called pet insurance, a huge number of photographs of an animal and a medical history of the animal after the time of imaging of the animal are accumulated, and the present inventors have examined whether the above-described problem can be solved by using such data. As a result, with artificial intelligence having learned the record of the photographs and the medical history of the animal as training data, it is found that a prediction model for assessing whether the animal may contract a disease in the future from the photographs of the animal can be created to complete the invention.

In other words, the invention includes the following [1] to [6]:
[1] A disease prediction system including: a reception means for receiving an input of a facial image of an animal excluding a human; and an assessment means for predicting and determining whether the animal may contract a disease from the facial image of the animal input to the reception means by using a learned model; characterized in that the learned model is a learned model that performs learning by using facial images of animals excluding humans and the presence or absence of a contracted disease within a predetermined period from the time of imaging the animals as training data, and inputs a facial image of an animal and outputs a predictive assessment regarding whether the animal may contract a disease.
[2] The disease prediction system according to [1], wherein the animal is a dog.
[3] The disease prediction system according to [1] or [2], wherein the input image is an image obtained by imaging the face of the animal from the front.
[4] An insurance fee calculation system for inputting a facial image of an animal to be in coverage of insurance into the disease prediction system according to any one of [1] to [3] and determining an insurance fee of the animal in accordance with the output prediction of contraction of a disease.
[5] A method for creating a disease prediction model, characterized in that artificial intelligence learns by using facial images of animals excluding humans and the presence or absence of a contracted disease within a predetermined period from the time of imaging the animals as training data.
[6] A disease prediction method including the steps of: preparing a facial image of an animal excluding a human; and inputting the facial image to a learned model and outputting a prediction regarding whether the animal may contract a disease within a predetermined period from the input facial image of the animal by using the learned model; characterized in that the learned model is a learned model that performs learning by using facial images of animals excluding humans and the presence or absence of a contracted disease within a predetermined period from the time of imaging the animals as training data, inputs a facial image of an animal, and outputs a prediction regarding whether the animal may contract a disease within a predetermined period.

### EFFECTS OF THE INVENTION

The invention makes it possible to provide a disease prediction system for assessing, by way of a simple method, the possibility that an animal may contract a disease in the future, and the like.

### BRIEF DESCRIPTION OF THE DRAWINGS

FIG. 1 illustrates an example of a suitable facial image of an animal.
FIG. 2 illustrates an example of a suitable facial image of an animal.
FIG. 3 is a schematic diagram illustrating one embodiment of an insurance fee calculation system according to the present invention.
FIG. 4 is a facial image (photograph) of an animal used for learning of the embodiment.
FIG. 5 is a facial image (photograph) of an animal used for learning of the embodiment.
FIG. 6 is a facial image (photograph) of an animal used for learning of the embodiment.
FIG. 7 is a table illustrating the number of images used for learning of the embodiment and a test result.

### MODE FOR CARRYING OUT THE INVENTION

### [Disease Prediction System]

The disease prediction system of the present invention includes: a reception means for receiving an input of a facial image of an animal excluding a human; and an assessment means for assessing and outputting a prediction of contraction of a disease of the animal from the facial image of the animal input to the reception means by using the learned model.

### [Reception Means]

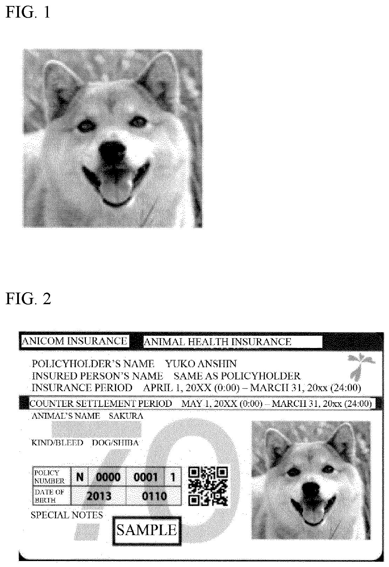

The reception means of the present invention is a means for receiving an input of a facial image of an animal for which contraction of a disease is to be predicted. Examples of the animal include a dog, a cat, a rabbit, a ferret, and the like. An image receiving method may be any method such as scanning, input of image data, and transmission. Although the format of a facial image is not particularly limited, it is preferable that the facial image is a photograph obtained by imaging the face of an animal from the front, and a close-up facial photograph of an animal as shown in FIG. 1 is more preferable. An example of such a photograph is a photograph of a driver's license of a human. An image used for a health insurance certificate of an animal, as shown in FIG. 2, is also preferable. The image may be black-and-white, gray scale or color. A partial image of the face of an animal, an image in which the shape is edited by image editing software, an image in which a plurality of animals is imaged, an image in which the face is too small to discriminate the eyes and ears, or an unclear image is not preferable. It is preferable, for example, that the images are normalized and have the same degree of resolution.

### [Assessment Means]

The assessment means of the present invention includes a learned model that performs learning by using a facial image of an animal excluding a human and the fact of contraction of a disease within a predetermined period from the time of imaging the animal as training data, inputs a facial image of an animal, and outputs a prediction regarding whether the animal may contract a disease.

Artificial Intelligence (AI) is preferable as the learned model. Artificial Intelligence (AI) refers to software or a system that uses a computer to mimic intellectual work performed by human brains, and specifically refers to a computer program or the like that recognizes a natural language used by a human or performs logical inference or learns from experience. Artificial Intelligence may be either a general-purpose type or a specialized type, and may be any one of a deep neural network, a convolutional neural network, and the like, and publicly available software can be used.

In order to create a learned model, artificial intelligence is programmed to perform learning by using the training data. The learning may be either machine learning or deep learning, but deep learning is preferable. Deep learning is an advanced extension of machine learning, and is characterized in that a feature value is automatically found.

A learning method for creating a learned model is not particularly limited, and publicly available software can be used. For example, DIGITS (the Deep Learning GPUTraining System) disclosed by NVIDIA can be used. For example, artificial intelligence may be trained by the well-known Support Vector Machine Method published in "An introduction to support vector machine" (Kyoritsu Shuppan Co.) or the like.

The training data for learning include a facial image of an animal and the presence or absence of a contracted disease, that is, whether the animal has contracted a disease from the time of imaging the facial image within a predetermined period, preferably within three years, more preferably within two years, and more preferably within one year. The facial image of the animal as the training data is the same as the facial image described in the reception method. The information on whether the animal has contracted a disease can be obtained, for example, from an animal hospital or an insured owner as a fact of an insurance claim (also referred to as an "accident"). In other words, when the animal is a beneficiary of pet insurance, the animal hospital or the owner (policyholder of the pet insurance) makes a claim to an insurance company together with the fact that the animal has contracted a disease. The insurance company thus knows that the animal has contracted a disease. On the other hand, when any payment of the insurance money is not claimed from the preparation of the facial image to the lapse of a predetermined time, it can be assessed that the animal as a beneficiary of pet insurance has not contracted a disease during the time period.

A learned model may be created for an individual disease, or for a plurality of diseases as a group. When a learned model is created for each disease, concerning an animal that has contracted a specific disease, a facial image imaged within a predetermined period from contraction of the disease, the fact of contraction of the disease, and as a control, a facial image of an animal that has not contracted a disease within a predetermined period from the time of imaging of a facial image and the fact that the animal has not contracted the disease during the predetermined period, are learned as training data. When a plurality of diseases is collectively learned, a plurality of types of training data may be prepared as training data in such a way that, as training data, an animal that has contracted a disease and a facial image thereof imaged within a predetermined period from its contraction of the disease, an animal that has contracted another disease different from the disease and a facial image thereof imaged within a predetermined period from its contraction of the disease, and an animal that has contracted a disease and a facial image thereof imaged within a predetermined period from its contraction of the disease, and so on.

### [Disease]

The type of a disease as targeted by the invention is not particularly limited, and examples thereof include an ophthalmic disease, an otological disease, and a dermatological disease. Ophthalmic diseases include conjunctivitis, eye mucus, keratitis, and corneal ulcer/erosion, lacrimation, cataract, and glaucoma. Otological diseases include otitis externa and otitis media. Dermatological diseases include dermatitis, atopic dermatitis, and pyoderma.

### [Output]

When receiving a facial image of an animal as input information, the assessment means of the invention performs predictive assessment, by using a learned model, regarding whether the animal may contract a disease within a predetermined period, preferably within 3 years, more preferably within 2 years, and more preferably within one year from the time of imaging of a facial image.

The format of the output is not particularly limited, and the predictive assessment can be output by displaying, for example on the screen of a personal computer, a message such as "Likely to contract a disease within one year from now on" or "Unlikely to contract a disease within one year from now on".

When a learned model specified for an individual disease is created, predictive assessment is performed regarding whether the animal has contracted a disease within a predetermined period after image reception. When a learned model including a plurality of diseases is created, predictive assessment is performed regarding whether a specific disease may be contracted among those included in the training data within a predetermined period after image reception.

The disease prediction system of the invention may separately include an output means for receiving an assessment result from the assessment means and outputting an assessment result.

### [Insurance Fee Calculation System]

The insurance fee calculation system of the invention inputs a facial image of an animal to be in coverage of insurance to the disease prediction system, and determines the insurance fee of the animal in accordance with the prediction of contraction of a disease that has been output. Information such as the kind, age, gender, and weight of the animal may be used in addition to the prediction of contraction of the disease.

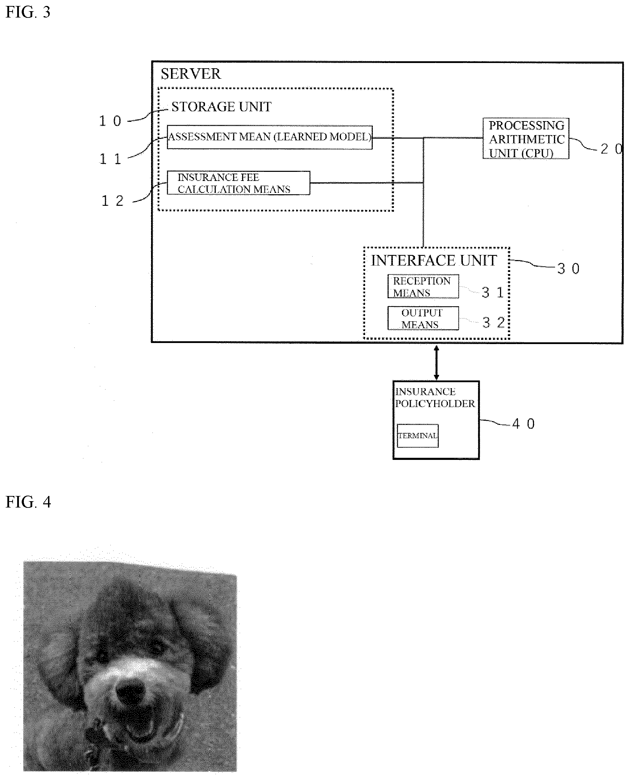

Hereinafter, an embodiment of the insurance fee calculation system of the invention will be described with reference to FIG. 3.

Referring to FIG. 3, a terminal 40 is a terminal used by an insurance policyholder (user). The terminal 40 may be, for example, a personal computer or a tablet terminal. The terminal 40 includes a processing unit such as a CPU, a storage unit such as a hard disk, a ROM or a RAM, a display unit such as a liquid crystal panel, an input unit such as a mouse, a keyboard or a touch panel, and a communication unit such as a network adapter.

An insurance policyholder accesses to a server from the terminal 40, inputs and transmits information such as a facial image (photograph) of an animal to be in coverage of insurance, a kind, a breed, an age at the time of imaging, a weight, and a past medical history of the animal.

The insurance policyholder receives a disease prediction result and an insurance fee calculation result stored in the server by accessing to the server from the terminal 40.

While the server includes a computer in the embodiment, any device equipped with a function according to the invention may be used as a server.

A storage unit 10 includes, for example, a ROM, a RAM, a hard disk, and the like. The storage unit 10 stores an information processing program for operating each unit of the server. In particular, an assessment means (learned model) 11, and, as appropriate, an insurance fee calculation means 12 are stored. When the insurance fee calculation system is to be configured as a disease prediction system that simply outputs a prediction of contraction of a disease without aiming to calculate an insurance fee, the insurance fee calculation means 12 may be omitted.

The assessment means (learned model) 11 uses as an input a facial image of an animal, input by an insurance policyholder, which animal is to be covered by insurance as described above, and outputs a prediction regarding whether an animal included in the image may contract a specific disease within a predetermined period (for example, within one year) from the time of imaging or input of the facial image. The assessment means (learned model) 11 in the embodiment includes, for example, a deep neural network or a convolutional neural network.

The insurance fee calculation means 12 is software for calculating the insurance fee of the animal from a prediction of contraction of a disease output by the assessment means 11, and information such as the kind, bleed, age at the time of imaging, weight and past medical history of the animal as input by the insurance policyholder. For example, the software is software for classifying the insurance fee into a grade in accordance with the kind, bleed, age at the time of imaging, weight and past medical history of the animal, and finally calculating a final insurance fee by correcting the grade by taking into account the prediction of contraction of the disease output by the assessment means 11.

The insurance fee calculation means 12 and the assessment means (learned model) 11 may be a single piece of software.

A processing arithmetic unit 20 predicts contraction of a disease and calculates an insurance fee by using the assessment means (learned model) 11 and the insurance fee calculation means 12 that are stored in the storage unit.

An interface unit (communication unit) 30 includes a reception means 31 and an output means 32, receives a facial image of an animal and other information from a terminal of an insurance policyholder, and outputs a prediction of contraction of a disease and a calculation result of an insurance fee to the terminal of the insurance policyholder.

The insurance fee calculation system of the embodiment allows an insurance policyholder to create a health insurance card of a pet by uploading to a server a photograph to be used to create the health insurance card of the pet, and at the same time, obtain the insurance fee of the pet and a prediction regarding contraction of a disease in the future.

### EXAMPLES

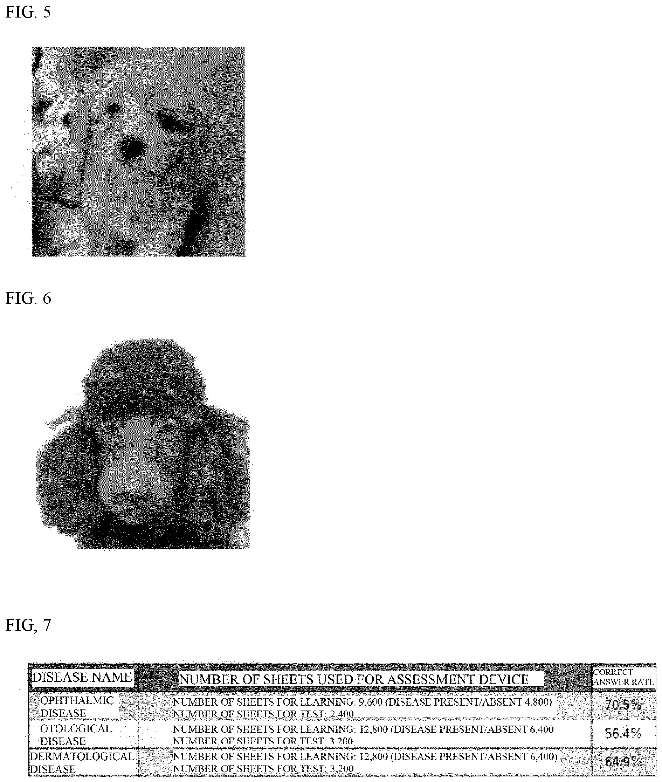

As many sheets as those in the table of FIG. 7 of face photographs of toy poodles (as an example, color photographs of FIG. 4 to FIG. 6, all in 256 x 256 pixels) are used to perform deep learning, and a learned model is created.

GoogleNet is used as artificial intelligence (neural network), and NVIDIA DIGITS Ver 3.0.0 is used as learning software. Deep learning is performed by labeling the photographic data depending on the presence or absence of a contracted disease.

Concerning an ophthalmic disease, 4800 sheets of images of toy poodles that have contracted an ophthalmic disease within one year from the time of imaging are used as photographs for training data, and a learned model A is created by using 4800 sheets of photographs of toy poodles that have not contracted an ophthalmic disease within one year.

A test is conducted, for the created learned model A, by using 2400 sheets among 9600 photographs used for learning. The correct answer rate of the assessment of the presence or absence of an ophthalmic disease, that is, the rate of the sum of the number of photographs of animals assessed as "disease-contracted" that have actually contracted a disease and the number of photographs of animals assessed as "disease-not-contracted" that have not actually contracted a disease within one year, to the total number of photographs is 70.5%.

Concerning an otological disease, 6400 sheets of photographs of toy poodles that have contracted an otological disease within one year from the time of imaging and 6400 sheets of photographs of toy poodles that have not contracted an otological disease within one year are used as photographs for training data, and a learned model B is created.

A test is conducted, for the created learned model B, by using 3200 sheets among 12800 photographs used for learning. The correct answer rate of the assessment of the presence or absence of an otological disease is 56.4%.

Concerning a dermatological disease, 6400 sheets of photographs of toy poodles that have contracted a dermatological disease within one year from the time of imaging and 6400 sheets of photographs of toy poodles that have not contracted a dermatological disease within one year are used as photographs for training data, and a learned model C is created.

A test is conducted, for the created learned model C, by using 3200 sheets among 12800 photographs used for learning. The correct answer rate of the assessment of the presence or absence of a dermatological disease is 64.9%.

The learned models A to C created above are assessed by using images of toy poodles that have not been used as training data.

Concerning an ophthalmic disease, 4066 sheets of images of toy poodles that have not been used as training data are used, and 2033 sheets of images of toy poodles are assessed to have not contracted an ophthalmic disease ("no accident"), and 2033 sheets of images of toy poodles are assessed to have contracted an ophthalmic disease within one year from the time of imaging ("accident"). The learned model A has a correct answer rate of 70.1% as a prediction of a disease of a toy poodle.

Concerning an otological disease, 4000 sheets of images of toy poodles that have not been used as training data are used. Of these, 2000 sheets of images of toy poodles are assessed to have not contracted an otological disease ("no accident"), and 2000 sheets of images of toy poodles are assessed to have contracted an ophthalmic disease within one year from the time of imaging ("accident"). The learned model B has a correct answer rate of 55.0% as a prediction of a disease of a toy poodle.

Concerning a dermatological disease, 4000 sheets of images of toy poodles that have not been used as training data are used. Among these, 2000 sheets are images of toy poodles that have not contracted a dermatological disease ("no accident"), and 2000 sheets are images of toy poodles that have contracted a dermatological disease within one year from the time of imaging ("accident"). The learned model C has a correct answer rate of 63.9% as a prediction of a disease of a toy poodle.

## Claims

1. A disease prediction system comprising: a reception means for receiving an input of a facial image of an animal excluding a human; and an assessment means for predicting and determining whether the animal may contract a disease from the facial image of the animal input to the reception means by using a learned model; **characterized in that**
the learned model is a learned model that performs learning by using facial images of animals excluding humans and the presence or absence of a contracted disease within a predetermined period from the time of imaging the animals as training data, inputs a facial image of an animal, and outputs a predictive assessment regarding whether the animal may contract a disease.

2. The disease prediction system according to claim 1, wherein the animal is a dog.

3. The disease prediction system according to claim 1 or 2, wherein the input image is an image obtained by imaging the face of the animal from the front.

4. An insurance fee calculation system for inputting a facial image of an animal to be in coverage of insurance to the disease prediction system according to any one of claims 1 to 3 and determining an insurance fee of the animal in accordance with the output prediction of contraction of a disease.

5. A method for creating a disease prediction model for predicting and determining, from a facial image of an animal excluding a human, whether the animal may contract a disease within a predetermined period, **characterized by** inputting facial images of animals excluding humans and the presence or absence of a contracted disease within a predetermined period from the time of imaging the animals to a computer including artificial intelligence as training data and making the artificial intelligence learn the data.

6. A disease prediction method including the steps of:
preparing a facial image of an animal excluding a human; and
inputting the facial image to a learned model and outputting a prediction by a computer regarding whether the animal may contract a disease within a predetermined period from the input facial image of the animal using the learned model; **characterized in that**
the learned model is a learned model that performs learning by using facial images of animals excluding humans and the presence or absence of a contracted disease within a predetermined period from the time of imaging the animals as training data, inputs a facial image of an animal, and outputs a prediction regarding whether the animal may contract a disease within a predetermined period.
